# EUROPEAN PATENT APPLICATION

(11) **EP 4 249 952 A1**
(43) Date of publication of application: **27.09.2023**
(21) Application number: 22315072.3
(22) Date of filing: 23.03.2022
(51) Int. Cl.: G01S 7/52, A61B 8/08, G01H 5/00

(54) **METHOD AND SYSTEM FOR EVALUATING AN ASSUMED WAVE PROPAGATION SPEED IN A MEDIUM**

(71) Applicant: SUPERSONIC IMAGINE, 13290 Aix en Provence (FR)
(72) Inventor: Zhang, Bo, 13109 Simiane-Collongue (FR)
(74) Representative: Paustian & Partner Patentanwälte mbB

(57) **Abstract**

The invention relates to a method of evaluating an assumed wave propagation speed in a medium, the medium being associated with spatio-temporal signal data, wherein the method comprises:
evaluating (e) the assumed wave propagation speed based on phase properties of the spatio-temporal signal data.

## Description

### FIELD OF THE DISCLOSURE

The present invention relates to methods and systems for evaluating an assumed wave propagation speed in a medium, in particular for medical imaging. In particular, the method is suitable for processing signal data of a medium scanned by a transducer device. For example, the method may be used in a device such as for instance an ultrasound system.

### BACKGROUND OF THE INVENTION

It is known to use a plurality of transducer elements or transceivers (for example arranged as an array) for communication, imaging or scanning purposes, for example in the field of medical imaging, radar, sonar, seismology, wireless communications, radio astronomy, acoustics and biomedicine. One example comprises ultrasound imaging.

The aim of ultrasound imaging is to estimate the medium reflectivity. In a conventional ultrasound imaging method, an ultrasound transducer device (also referred to as an ultrasound probe) with a set of ultrasound transducer elements may be used. In the method, one or multiple transducers are used to transmit one or successively several ultrasound beam into a medium, corresponding to a transmission operation. Then, in a reception operation a set of backscattered echo signals are received from the medium by the set of transducer elements. In particular, each of the transducer elements converts a received echo signal into for example an electrical signal. The signal may further be processed by the ultrasound system. For example, they may be amplified, filtered digitalized and/or a signal conditioning operation may be carried out. The transducer elements may be arranged as a transducer array.

Conventionally, said signals are then transmitted to an image processing system. The received signals may be processed to generate image data of the scanned medium, for example using a beamforming method. Generally, beamforming may be understood as a signal processing technique conventionally used in sensor arrays for directional signal transmission or reception. This process is used to generate beamformed data. In other words: beamforming may be understood as a signal processing technique that is achieved by combining elements in an antenna array (for example an ultrasound transducer) in such a way that the combined signals form constructive interferences.

Processing of the received signals often includes assuming a wave propagation speed in the medium. Wave propagation speed inhomogeneities of the medium and inaccurate estimations of the wave propagation speed can lead to defocusing and increased clutter.

Traditionally, a predetermined value for an assumed wave propagation speed can be used, for example depending on the considered medium. For instance, one predetermined value may be used for soil, more specifically for different gravels or rocks, for a specific organ of human body, a specific kind of human tissue, or specific values can be used for muscles and fat, respectively.

Alternatively, a wave propagation speed for a medium can be determined by processing signals received from the medium.

Napolitano et al. describe in their article "Sound speed correction in ultrasound imaging", Ultrasonics 44 (2006) e43-e46, an algorithm that determines the sound speed that produces the best overall lateral image quality by analyzing the spatial frequency content in a single B-mode frame of channel data using images reconstructed using various trial sound speeds. The authors found that the metric produced by their algorithm correlated well with the observed best lateral image quality. This approach requires reconstructing B-mode images for different trial sound speeds by beamforming received signal data. The speed determination is based on the different B-mode images.

Imbault et al. describe in their article "Robust sound speed estimation for ultrasound-based hepatic steatosis assessment", Physics in Medicine & Biology 62 (2017) 3582, a speed of sound estimation method based on the assessment of spatio-temporal properties of the local speckle noise. Here, cross-correlations between backscattered echoes received by different transducers are evaluated and the sound speed is estimated by optimizing the spatial coherence of backscattered speckle noise.

Both methods described above rely on performing intricate mathematical operations, many iterations, in some cases on beamformed signal data.

Conventionally, evaluation of assumed wave propagation speeds in a medium may be a computationally intensive task, which may limit its use in real-time imaging context.

EP 3 632 330 A1 filed by the applicant of the present disclosure discloses a method for determining a target speed of sound inside a target region of a medium using an ultrasound imaging system. The method comprises the steps of determining a position of an interface in the medium, determining a first speed of sound of an intermediate region above the interface, and determining a target speed of sound inside a target region below the interface based on at least some of sensed signals and taking into account the position of the interface and the first speed of sound.

### SUMMARY OF THE DISCLOSURE

Currently, it remains desirable to overcome the aforementioned problems and in particular to provide a method and system of evaluating an assumed wave propagation speed in a medium, which advantageously is faster, more reliable, more robust, computationally less expensive and thus would require less processing power and being compliant with real time constraints.

Therefore, a method of evaluating an assumed wave propagation speed in a medium is provided. The medium is associated with spatio-temporal signal data, and the method comprises: (e) evaluating an assumed wave propagation speed based on phase properties of the spatio-temporal signal data.

An assumed wave propagation speed may be predetermined/preselected based on a theoretical model and/or on measured data and/or on prior clinical knowledge data, for example one or several lookup tables. The assumed wave propagation speed may for instance have a value within a predefined speed range. Said predefined speed range may depend on characteristics of the medium, for example the type of the medium. The assumed wave propagation speed may be a hypothesis and/or a candidate speed of an actual wave propagation speed. The term "actual wave propagation speed" may denote a physical propagation speed of waves in the medium, i. e. the speed at which a wave propagates through the medium. The actual wave propagation speed may depend on the type of the wave and on wave characteristics, for example on the wave frequency. The term "actual wave propagation speed" may refer to the physical propagation speed of waves having the same type and/or the same characteristics as, for example, those of waves transmitted into the medium and/or received from the medium for generation of the spatio-temporal signal data.

The wave may be a soundwave, for example an ultrasonic wave. The spatio-temporal signal data may be pre-beamformed spatio-temporal signal data.

The term "wave propagation speed in a medium" may refer to a wave propagation speed in subparts of the medium (for example a region of interest RoI) and/or to an average wave propagation for a medium. The wave propagation speed may refer to a global wave propagation speed. The wave propagation speed may refer to specific parts of the medium, such as for example pathological tissue, for example tumor tissue.

The "term wave propagation speed" may be understood to comprise the term "speed of sound", in case the waves are for example ultrasonic waves or other types of sound waves. The terms "wave propagation speed" and "speed of sound" may be used synonymously throughout this disclosure unless denoted differently.

The assumed wave propagation speed may be predetermined and/or preselected based on the medium, for example based on specific properties of the medium and/or based on the type of the medium, for example human tissue or predefined human organs to be considered. The assumed wave propagation speed may be an assumption of an actual wave propagation speed. The assumed wave propagation speed may be referred to and understood as an assumption of a wave propagation speed. The terms speed assumption and assumed speed may be used synonymously throughout this disclosure unless denoted differently.

Evaluating an assumed wave propagation speed may comprise determining a quantitative and/or qualitative measure of-the assumed wave propagation speed that may refer to the correspondence between the assumed wave propagation speed and an actual wave propagation speed in the medium. The quantitative and/or qualitative measure may also be referred to as a figure of merit. The quantitative and/or qualitative measure and/or the figure of merit may allow comparing different assumed propagation speeds (or propagation speed assumptions), in order to evaluate them, for example by comparing respective measures and/or respective figures of merit corresponding to the different assumed wave propagation speeds. Evaluation based on phase properties may be done based on and/or by comparing phase properties of individual signals from the plurality of the spatio-temporal signal data with each other.

The operation of evaluating (e) the assumed wave propagation speed may be based on signal-readout locations and/or the locations where signals are received. Spatio-temporal signal data may be read out and/or received at a plurality of locations. More in particular, the spatio-temporal signal data may comprise signal-readout locations and/or the locations where signals are received. Signal readout locations and/or the locations where signals are received may be located in the medium and/or on the medium, for example on a surface of the medium.

The spatio-temporal signal data may comprise spatial and temporal information and in particular (spatio-temporal) signal information.

An amplitude independent way of evaluating an assumed wave propagation speed may be provided. Examples of the present disclosure advantageously do not require costly computational operations such as beamforming data or computing correlation functions. Instead, according to examples of the present disclosure it becomes possible to evaluate an assumed wave propagation speed based on phase properties without complex data processing and optionally without referring to amplitude data and/or amplitude dependent data. Accordingly, the evaluations are advantageously faster, more reliable, more robust, computationally less expensive, and thus require less processing power. Due to the increased robustness, a decreased variance and thus an increased reproducibility can advantageously be achieved.

The present disclosure also allows for example to obtain much higher image quality and provide more reliable inputs to end user, for example when the method is used to estimate an actual wave propagation speed. The present disclosure may lead to a better medical exam when it is about medical usage of it.

A more accurately estimated actual wave propagation speed may also allow for better quantification. The wave propagation speed in a medium can have clinical meanings. For example, a fattier tissue has a lower wave propagation speed. Accordingly, lower wave propagation speeds can indicate a fatty liver disease in liver steatosis. An evaluated speed may also be used for other purposes than imaging, for example for analysing the tissue as a function of the evaluated speed. The evaluated speed and/or the estimated actual wave propagation speed may be used for example as a biomarker.

Hereby, more accurate assumed wave propagation speeds usually allow to build images with a higher quality, for example a higher spatial resolution and/or higher contrast/signal-to-noise ratio.

The spatio-temporal signal data may be or may comprise pre-beamformed spatio-temporal signal data and/or radiofrequency (RF) data. Pre-beamformed data may be data that have not been processed by beamforming and/or that will be processed by beamforming after evaluating the assumed wave propagation speed. The method may comprise beamforming after operation (e) of evaluating the assumed wave propagation speed. The pre-beamformed data may denote unprocessed data and/or measured data and/or raw data.

The spatio-temporal signal data may comprise a plurality of oscillating and/or periodic signals received from the medium and/or sections of the plurality of oscillating signals. The spatio-temporal signal data may be multi-channel data, wherein each channel comprises one signal. Each signal of the spatio-temporal signal data and/or the plurality of oscillating and/or periodic signals may comprise at least one oscillating signal component.

Sections of the plurality of oscillating signals may be time sections, for example signal data that fulfill a certain temporal condition and/or one or more instances in time of a respective signal. Time sections may for example be selected based on the assumed wave propagation speed.

The spatio-temporal signal data may be pre-recorded, for example the recording of the spatio-temporal signal data may have happened earlier in time. The recording of the spatio-temporal signal may have happened at a different location, for example in a hospital, while the spatio-temporal signal data may be used in operations performed at another location, for example in a computing device located in data center, in a cloud environment, and/or in a different city. The recording of the spatio-temporal signal data may not need to be comprised in the method. The recording of the spatio-temporal signal data may be comprised in the method. Examples of the present disclosure may provide signal post-processing techniques.

Phase properties may comprise any kind of phase properties. Especially, phase properties may comprise at least one of: Signs, phases, a sign proportion, phase intervals, proportions of phase evolution states, phase evolution states, and proportions of phase properties.

The "sign" may indicate whether a signal section (for example one or more instances in time of a signal) is positive or negative. A section of a signal may be one or more instances in time of a signal. In other words, the "sign" may be a binary value being "positive" or "negative". The term "signal section" may be understood as a signal fraction.

The term "sign proportion" may refer to the fraction of signals of a plurality of signals whose signs falls into a certain category, for example by fulfilling a certain criterion, for example being positive or negative, being ascending or descending, and/or combinations thereof. The term "phase intervals" may refer to phases of a plurality of signals being within specified phase intervals, for example between 0° and 180° or between 0° and 90° or between 0° and 45°. The plurality of signals may origin for example from different signal sources (for instance from different transducer elements). In case of only two categories (for example positive sign and negative sign), the term "proportion" may refer to the higher proportion. In case of more than two criteria, the term "proportion" may refer to the highest proportion of a distribution of the categories. The figure of merit may be a function of the category distribution. In other examples, the term "proportion" may refer to the lowest proportion of the respective proportions. The following is described assuming that the highest proportion is selected as "the" proportion. However, it is possible to use the lowest proportion instead. In such a case, the word "highest proportion" has to be replaced with "lowest proportion" wherever appropriate, as is readily apparent to the skilled person.

For example, phase evolution states may be defined based on derivatives of the spatio-temporal signal data, for example as a function of time. For example, phase evolution states may denote phase properties such as phases corresponding to "increasing signals" (for example a sinusoidal wave whose phase is between 0° and 90° or 270° and 360°), "decreasing signals" (for example a sinusoidal wave whose phase is between 90° and 270°), "positive sign increasing" (for example a sinusoidal wave whose phase is between 0° and 90°), "negative sign increasing" (for example a sinusoidal wave whose phase is between 270° and 360°), "positive sign decreasing" (for example a sinusoidal wave whose phase is between 90° and 180°), "negative sign decreasing" (for example a sinusoidal wave whose phase is between 180° and 270°), "positive sign" (for example a sinusoidal wave whose phase is between 0° and 180°), "negative sign" (for example a sinusoidal wave whose phase is between 180° and 360°), "minimal change of signal" (for example a sinusoidal wave whose phase is around 90° or around 270°), "maximum change of signal" (for example a sinusoidal wave whose phase is around 180° or 0°/360°), and various other categories readily apparent for the person skilled in the art, such as combinations of above categories or categories denoting selected phase intervals (for example a sinusoidal wave whose signal is between X° and Y°, wherein X and Y may be selected as appropriate). Phase evolution states may be pre-defined.

Using phase properties, for example sign proportions, may imply the advantage that a method of evaluating an assumed wave propagation speed is provided which may rely on the information of one instance in time of a signal. For example, one instance in time of a signal is sufficient to determine whether the sign of a signal is positive or negative. Examples of the present disclosure thus may provide a more robust method of evaluating an assumed wave propagation speed in a medium compared to other methods where knowledge of one instance in time of a signal is not sufficient.

Using evolution states may advantageously allow for a finer categorization of phase properties. This may allow to provide a more precise method of evaluating an assumed wave propagation speed and/or estimating an actual wave propagation speed. Using evolution states may require knowledge of respective series of signal data, for example temporal series, for example to determine whether a respective signal is increasing and/or decreasing. Such a method may be more complex and/or less robust. According to examples, broad categories such as sign properties and finer categories such as phase evolution states may be used in combination and/or subsequently. For example, broad categories such as sign proportions may be used in a first iteration and/or in first iterations, and finer categories, for example proportions of phase evolution states and/or phase evolution states may be used in subsequent iterations. Appropriate combination of different phase properties lies in the discretion of the skilled person and may provide methods that are robust, efficient, cheap, fast, and/or precise, optionally at the same time.

The method may further comprise:
determining (d) a figure of merit of the assumed wave propagation speed (i.e. of the speed assumption) based on the phase properties. Evaluating (e) the assumed wave propagation speed may be based on the figure of merit.

The figure of merit may be determined (d) by using a predefined function G, the function G being at least one of: a function G(x) that is symmetric about the axis x = 50%, configured to maximize at x=50% and minimize at x=0% and x=100% or to minimize at x=50% and maximize at x=0% and x=100, a bell shaped function, a normal distribution function, a negative Shannon entropy function (or more generally just a Shannon entropy).

Such a function G will provide the highest figures of merit if either all phase properties fall into a certain category or if none fall into a certain category, for example either all signs being positive or all signs being not positive (for example negative). The figure of merit may denote a purity of phase properties, for example a purity of signs, which is for example maximum if all signs fall into one category. For example, the figure of merit may be a purity measure of phase categories. The figure of merit may be based on the distribution of categories. For example, the figure of merit may be a negative Shannon entropy of the category distribution. The figure of merit may be maximized as the category distribution concentrates itself on a single category.

Phase properties, for example sign proportions or proportions of phase properties may be used as figures of merit, for example. The operation of determining (d) a figure of merit based on the phase properties may by performed simply by determining said phase properties. Determining (d) a figure of merit based on the phase properties may involve further operations based on the phase properties, for example calculations.

A figure of merit may be for example a quantitative and/or qualitative measure of the assumed speed that may refer to the correspondence between the assumed wave propagation speed and an actual wave propagation speed in the medium, as described above. A figure of merit may also be referred to as an evaluation value. The figure of merit may allow comparing different assumed propagation speeds (or propagation speed assumptions), in order to evaluate them. The figure of merit may for example be an entropy, for example more particularly a negative Shannon entropy, of phase properties, for example an entropy of sign proportions, for example an entropy of the ratio and/or the proportion of positive signs and/or negative signs.

The operation of determining (d) a figure of merit of the spatio-temporal signal data using a predefined function G may be performed before the operation of evaluating (e) the assumed wave propagation speed based on phase properties of the spatio-temporal signal data.

The spatio-temporal signal data may be associated with a predefined spatial region in the medium. For example, the spatio-temporal signal data may be associated with a pre-selected spatial region in the medium. The spatial region may correspond to one pixel and/or a plurality of pixels in beamformed image data corresponding to the medium. The pre-selected region may for example constitute a reflector in the medium. The spatio-temporal signal data may be associated with a predefined spatial region in the medium such that the spatio-temporal signal data comprise sections, for example time sections, of the spatio-temporal signal data.

The method may further comprise: determining (c) a subset of the spatio-temporal signal data as a function of a predefined spatial region in the medium and the assumed wave propagation speed. The subset may be determined based on the assumed wave propagation speed. The assumed wave propagation speed may be evaluated based on the subset, for example the assumed wave propagation speed may be evaluated by only considering spatio-temporal signal data of the respective subset. The operation of determining (c) a subset of the spatio-temporal signal data may be performed prior to the operations of evaluating (e) the assumed wave propagation speed and/or determining (d) a figure of merit of the spatio-temporal signal data.

The subset may comprise sections of the oscillating signals. The sections may be selected with respect to each other as a function of the spatial region and/or the assumed wave propagation speed, optionally in relation to the locations where the signals are received and/or the signal-readout locations. Selecting the sections may comprise shifting the sections in time and/or delaying the sections. The sections may be shifted and/or delayed relatively to each other depending on the selected spatial region. The sections may be delayed and/or shifted computationally. The sections may be delayed adaptively. The terms shifting and delaying may be used synonymously throughout this disclosure unless denoted differently.

Hence, phase properties of the spatio-temporal signal may be correlated to each other. Phase properties may be determined along each accordingly delayed wavefronts for a selected spatial region.

For example, the time sections of different signals of the plurality of signals may be delayed according to time intervals that are selected as a function of the spatial region and/or on the assumed wave propagation speed and/or signal-readout locations and/or the locations where signals are received, for example based on an assumed propagation time differences of a signal arriving from a spatial region at respective different signal readout locations. In such a case, the time intervals of respective signal sections after shifting of one or more from the plurality of oscillating signals may correspond to each other. Alternatively or in addition, time sections of respective ones of the plurality of oscillating signals may be selected such that time intervals of respective ones of the plurality of oscillating signals are time shifted with respect to each other, as a function of the spatial region and/or the assumed wave propagation speed and/ or the signal-readout locations and/or the locations where signals are received, for example based on propagation time differences, corresponding to the assumed wave propagation speed, between respective signal readout locations. Alternatively, or in addition, time sections may be selected based on respective distances between the spatial region and respective signal readout locations and/or based on the respective signal-readout locations and/or the respective locations where signals are received. For example, signals having the same distance, for example the same pulse-echo distance, from the spatial region may not be delayed with respect to each other and/or the time stamps of the respective time intervals may correspond to each other. The operation (c) of determining a subset of the spatio-temporal signal data may be based on the geometry of a plurality of transducers elements, for example a transducer array, used for receiving the spatio-temporal signal data.

The method may be iterated, in parallel or in series, for a plurality of subsets, each one being associated with a different spatial region in the medium, and the assumed wave propagation speed may be evaluated based on figures of merit of the plurality of subsets, for example based on averaging and/or combining respective figures of merit. The method may be iterated, in parallel or in series, for a plurality of (optionally predefined) spatial regions in the medium, and the assumed wave propagation speed may be evaluated based on figures of merit of the plurality of respective corresponding subsets, for example based on averaging and/or combining respective figures of merit.

The method may be repeatedly carried out for a plurality of spatial regions in the medium, in parallel or in series, and/or operations (c) and (d) may be repeated for a plurality of spatial regions in the medium, in parallel or in series. The method may comprise: averaging and/or combining respective figures of merit.

The assumed wave propagation speed may be evaluated based on figures of merits of selected subsets whose figures of merit fulfill certain criteria, for example being above a predefined threshold, and/or the assumed wave propagation speed may be evaluated based on weighted figures of merit, which may for example be weighted as a function of the respective figures of merit and/or which may be weighted according to a predefined weighting function. Criteria, for example thresholds, may be adapted in each iteration and/or may not be adapted. Thresholds may for example be applied in each iteration or only in some iterations.

For example, for uncorrelated signals, a uniform distribution of phase properties may be expected, for example 50 % of the signals exhibiting a positive sign while 50 % of the signals exhibiting a negative sign. In case of the spatial region having little or no reflectance, received signals may be largely uncorrelated, for example noisy. Phase properties of signals related to spatial regions having little or no reflectance may not be well suited to evaluate an assumed wave propagation speed in the medium. Only considering subsets whose figures of merit are above a certain threshold may allow to advantageously consider only the "meaningful" subsets and may thus improve the quality of the evaluation of the assumed wave propagation speed and decrease the computational effort and the duration of corresponding computational operations.

The method may be carried out for a plurality of different assumed wave propagation speeds. The method may optionally comprise: estimating (g) an actual wave propagation speed in the medium, optionally based on the assumed wave propagation speed having the highest figure of merit and/or the best figure of merit and/or the best evaluation of the assumed wave propagation speeds. If the phase properties on which the operation of evaluating (e) an assumed wave propagation speed is based are sign proportions, for example as a figure of merit, the method may comprise: estimating an actual wave propagation speed in the medium based on the assumed wave propagation speed having the highest sign proportion. The method may also comprise: estimating an actual wave propagation speed in the medium based on the assumed wave propagation speed having the lowest sign proportion. The operation of estimating (g) an actual wave propagation speed may be performed after operations (c), (d), and (e).

Respective ones of the plurality of assumed wave propagation speeds may be assumed based on a theoretical model and/or on measured data. The assumed wave propagation speeds may be assumed based on the medium, for example based on specific properties of the medium and/or based on the type of the medium, for example human organ or human tissue. The plurality of assumed wave propagation speeds may be chosen according to nested intervals. The plurality of assumed wave propagation speeds may be chosen from a predefined speed range.

The method may comprise: adjusting (f) the assumed propagation speed, optionally as a function of the figure of merit, and/or iterating the method, optionally such that the figure of merit is increased. The figure of merit may for example not need to be increased in every single iteration, but may for example increase overall across several iterations. The respective figures of merit may be used as a guidance for the adjusting (f). The method may comprise in each iteration an adjusted wave propagation speed. The operation of adjusting (f) the assumed propagation speed may be performed after operations (c), (d), (e) and prior to performing operation (g).

The method may comprise operation (f) only once and/or the method may be iterated only once. The method may comprise operation (f) more than once and/or the method may be iterated more than once. The plurality of assumed speeds may be refined after iterations, and the method may be iterated afterwards.

Operation (f) may allow to, optionally incrementally, reduce the difference between an actual wave propagation speed and an assumed wave propagation speed. Known optimization algorithms such as the Levenberg-Marquardt algorithm and/or the Gauss-Newton algorithm may be used during operation (f). The assumed wave propagation speed may be adjusted according to nested intervals. The assumed wave propagation speed may be adjusted according to a predefined speed range.

The wave propagation speed may be adjusted as a function of the figure of merit determined before, optionally in order to optimize the speed assumption and/or the assumed wave propagation speed, for example by decreasing the difference between an assumed wave propagation speed and an actual wave propagation speed.

Evaluating (e) the assumed wave propagation speed may comprise comparing the figure of merit with a predefined threshold. The method may be iterated until the figure of merit is above a predefined threshold and/or satisfies a certain threshold. The method may comprise estimating (g) an actual wave propagation speed in the medium based on the assumed wave propagation speed and/or the adjusted assumed wave propagation speed having the highest figure of merit and/or the best figure of merit.

If the figure of merit is above a predefined threshold already after evaluating (e) an assumed wave propagation speed once, an actual wave propagation speed may be estimated based on the assumed wave propagation speed without further iterations, i. e. based on a single assumed wave propagation speed.

A method optionally comprising operation (f) may be performed for a plurality of spatial regions, and a plurality of adjusted assumed wave propagation speeds may be obtained, optionally corresponding to the plurality of spatial regions. Estimating an actual wave propagation speed may be based on the plurality of adjusted assumed wave propagation speeds. For example, the plurality of adjusted assumed wave propagation speeds may be averaged, optionally with weights, optionally by excluding one or more of the adjusted assumed wave propagation speeds for example based on thresholds for respective figures of merits, for example predefined thresholds, and/or by excluding assumed adjusted wave propagation speeds being outliers and/or whose figures of merit being outliers.

The plurality of signs may be received by a respective plurality of transducer elements.

The method may comprise, optionally before determining (c) the subset and/or determining (d) the figure of merit: transmitting (a) a pulse into the medium and/or receiving (b) in response the plurality of signals from the medium.

The pulse may be an ultrasonic pulse. The plurality of signals may be a plurality of ultrasonic signals.

The disclosure may comprise a method for estimating a wave propagation speed in a medium based on a method of evaluating a plurality of assumed wave propagation speeds.

The disclosure may refer to a computer program comprising computer-readable instructions which when executed by a data processing system cause the data processing system to carry out a method according to any examples of the present disclosure.

The disclosure may refer to a system of evaluating an assumed wave propagation speed in a medium. The medium may be associated with spatio-temporal signal data. The system may comprise a processing unit configured to evaluate the assumed wave propagation speed based on the spatio-temporal signal data and/or based on phase properties of the spatio-temporal signal data. The system may furthermore be configured to perform any of the method features or operations described above.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

### BRIEF DESCRIPTION OF THE DRAWINGS

Fig. 1 shows a schematic drawing showing an ultrasound system according to examples of the present disclosure; and
Fig. 2 shows a diagram of a method of evaluating an assumed wave propagation speed in a medium according to the present disclosure, the method may be implemented in the system of Fig. 1;
Fig. 3 shows a first example of evaluating an assumed wave propagation speed based on phase properties, which may for example be implemented in the system of Fig. 1 and/or according to the method of Fig. 2;
Fig. 4 shows figures of merit determined as a function of a plurality of assumed wave propagation speeds according to an example of the present disclosure; and
Fig. 5 shows a plurality of different phase properties that may be used according to examples of the present disclosure.

### DESCRIPTION OF THE DRAWINGS

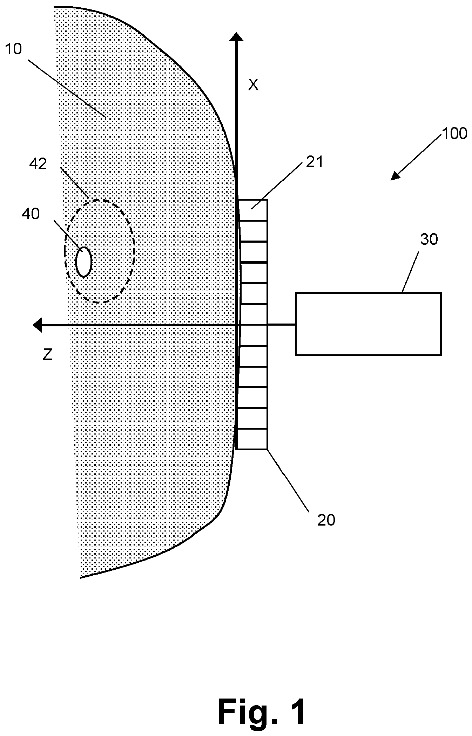

Reference will now be made in detail to examples of the disclosure, examples of which are illustrated in the accompanying drawings. Wherever possible, the same reference numbers will be used throughout the drawings to refer to the same or like parts. The system 100 shown on Fig. 1 is adapted for evaluating an assumed wave propagation speed in a medium 10, for instance living tissues and in particular human tissues of a person, for example a sporty professional and/or a patient. The system may include for instance:
- A plurality of transducer elements 21. The transducer elements may be configured to transmit (a) a pulse into the medium and/or to receive (b) a plurality of signals from the medium, optionally in response to transmitting (a) the pulse into the medium. A transducer array comprising a plurality of transducer elements 21 may be used. For example, a linear array 20 may be provided typically including a few tens of transducer elements (for instance 100 to 300) juxtaposed along an axis X (horizontal or array direction X) as already known in usual probes. In this example, the array 20 is adapted to perform a bidimensional (2D) imaging of the medium 10, but the array 20 could also be a bidimensional array adapted to perform a 3D imaging of the medium 10. The transducer array 20 may also be a convex array including a plurality of transducer elements aligned along a curved line. The same transducer element(s) may be used to transmit a pulse and receive the response, or different transducer elements are used for transmission and reception. There may be one or more emitting transducer elements and a plurality of receiving transducer elements. In a further alternative, only a single transducer element may be used which receives a plurality of signals which have different spatial properties (for example originating from different spatial regions). The transducer element may for instance be electronically or physically moveable;
- an electronic bay 30 controlling the transducer array and acquiring signals therefrom.

The system may further include a microcomputer (not shown) for controlling the electronic bay 30 and/or for example for sending data to a server, to an artificial intelligence (AI) entity, to a dedicated workstation, presenting data, displaying/viewing images obtained from the electronic bay (in a variant, a single electronic device could fulfil all the functionalities of the electronic bay 30 and the microcomputer).

The transducer elements 21 may comprise piezo-crystals and/or other components that may be configured to generate and/or record and/or receive signals. The terms transducer and transducer elements may be used synonymously throughout this disclosure unless denoted differently.

Transducer elements 21 may be configured to generate and/or record and/or receive signals, optionally ultrasonic signals. Transducer elements 21 and/or electronic bay 30 and/or the microcomputer may be configured to determine phase properties of spatio-temporal signal data.

The axis Z on figure 1 is an axis perpendicular to the axis X, for example in the depth direction of the examined medium. This direction is designated in the present document as a vertical or axial direction.

The medium 10 may comprise a spatial region 40. The spatial region 40 may be predefined and/or may be selected during operation of methods according to examples of the present disclosure. The medium 10 may comprise a plurality of spatial regions 40.

The medium 10 may optionally comprise one or more regions of interest 42. Spatial regions 40 optionally may be comprised only in one or more regions of interest 42. Spatial regions 40 may be located in the medium at various positions not necessarily restricted to specific regions of interest 42. The whole medium or a part of the medium may be the region of interest 42. For example, only regions corresponding to regions in beamformed image data corresponding to the medium and/or the region corresponding to the entire beamformed image data may be regions of interest 42. For example, in case of a tumor, it may be most interesting to evaluate an assumed wave propagation speed in the supposed tumor region while the remaining tissue may be of minor interest.

The system herein disclosed is a device for ultrasound imaging, the transducer elements are ultrasound transducer elements, and the implemented method evaluates an assumed wave propagation speed in the medium 10 based on phase properties of spatio-temporal signal data. The medium 10 is associated with the spatio-temporal signal data. The method optionally may produce ultrasound images of the medium 10 and/or of spatial regions 40 of the medium 10 and/or may send data to a dedicated server or working station.

However, the system may be any imaging or sensor device using other waves than ultrasound waves (for example waves having a wavelength different than an ultrasound wavelength and/or waves being no sound waves), the transducer elements and the electronic bay components and related elements being then adapted to said waves.

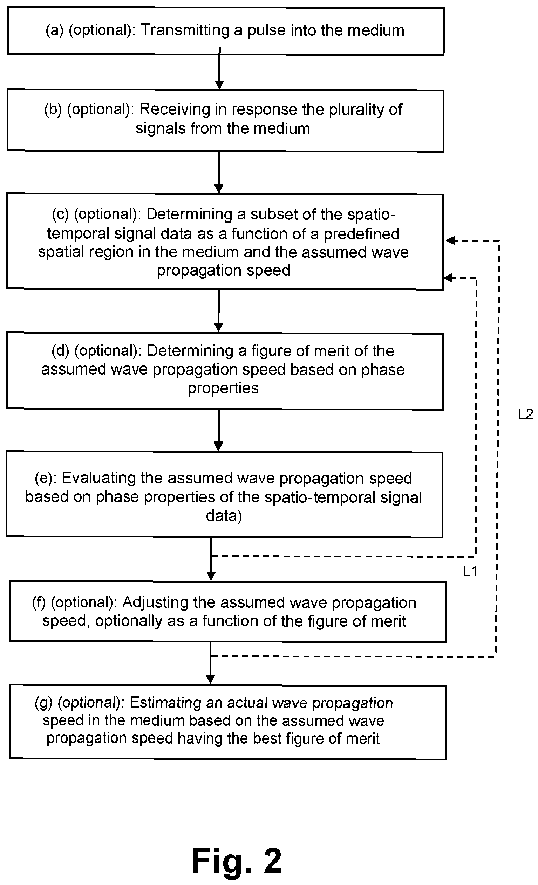

Fig. 2 shows a diagram of a method of evaluating an assumed wave propagation speed in a medium according to the present disclosure. The method may be implemented in the system of Fig. 1.

The method may comprise an optional operation of transmitting (a) a pulse into the medium. For example, the transmission operation may comprise insonification of the medium with a cylindrical wave that focuses on a given point and/or plane waves of different angles. More in particular, during the transmission operation a plurality of ultrasonic waves may be transmitted into a spatial region 40.

Generally, in the present disclosure a pulse may correspond to an acoustic and/or electrical signal emitted by a transducer element. The pulse may for example be defined by at least one of: the pulse duration, the frequency of the resulting wave, the number of cycles at the given frequency, the polarity of the pulse, etc. A wave may correspond to the wavefront generated by one or several transducer elements (i.e. by respectively emitted pulses). The wave may be controlled by means of emission delay between the different used transducer elements. Examples comprise a plane wave, a focused wave and a divergent wave. A beam may correspond to the physical area insonified by the wave (for example in the medium). Hence, the beam may be related to the wave but may have less or no temporal notion. For example, it may be referred to a beam when the depth of field of a focused beam is of interest.

In an optional operation (b), a plurality of signals may be received, optionally in response, from the medium by the plurality 20 of transducer elements 21. The plurality of signals may comprise backscattered echoes of the transmission of operation (a). The response sequence may also be referred to as spatio-temporal data and/or signal data, in particular ultrasound signal data and/or RF and/or IQ signal data. The signal data may be in the time domain, more in particular in a spatio-temporal domain, as for example described in more detail below. In one example, the response sequence may be processed by bandpass filtering, in order to keep only one or several frequency ranges.

It is noted that operations (a) to (b) are optional, as they may also be carried out by any other system than the system used for operation (e) and optional operations (c), (d), (f), (g) and/or at another time. Data may also be provided by other functionalities such as simulation devices, insonification on a phantom, etc. It is also possible that the spatio-temporal signal data are pre-stored, and for example provided by/read on a data storage, a communication interface, etc.

In an optional operation (c), a subset of the spatio-temporal signal data may be determined as a function of a predefined spatial region 40 in the medium 10 and as a function of the assumed wave propagation speed. For example, data in the subset corresponding to a specific spatial region 40 in the medium 10 may be determined based on the assumed propagation time of a signal between the spatial region 40 and respective transducer elements 10. The assumed propagation times may be determined based on the location of the spatial region 40 and the location of the plurality of transducers and/or each respective transducers, optionally in relation to the location of the spatial region 40, and the assumed wave propagation speed. Determining a subset of the spatio-temporal signal data as a function of a predefined spatial region 40 in the medium 10 and as a function of the assumed wave propagation speed may be based on the geometry of the plurality 20 of transducers 21. It is noted that the spatial region may be associated with a pixel or a plurality of pixels in beamformed image data. The optional beamforming process may be done in an optional operation after operation (g).

As described above, spatial regions 40 optionally may be comprised only in one or more regions of interest 42. Spatial regions 40 may be located in the medium at various positions not restricted to specific regions of interest 42. The whole medium or a part of the medium may be the region of interest 42. For example, only regions corresponding to regions in beamformed image data corresponding to the medium and/or the region corresponding to the entire beamformed image data may be regions of interest 42. For example, in case of a tumor, it may be most interesting to evaluate an assumed wave propagation speed in the supposed tumor region while the remaining tissue may be of minor interest.

Methods of the present disclosure may also be used for estimating a spatial extent of a region of interest, for example a tumor. One or more regions of interest may be indicated by a user and/or selected automatically (for example by an artificial intelligence), optionally based on historic data that for example may have been generated during earlier examinations.

In an optional operation (d), a figure of merit of an assumed wave propagation speed may be determined, based on the phase properties. For example, an entropy of phase properties may be computed, for example a negative Shannon entropy of sign proportions.

In operation (e), the assumed wave propagation speed is evaluated based on phase properties of the spatio-temporal signal data. The operation (e) may be based on the figure of merit optionally determined in operation (d). Operation (e) may be performed after one or several iterations of operation (d) (for example, each operation (d) may be carried out for a different subset).

It may be desirable to evaluate an assumed wave propagation speed as a function of a plurality of predefined spatial regions. For example, some spatial regions 40 may not constitute good reflectors and may therefore not be well suited for evaluating an assumed wave propagation speed. Furthermore, using a plurality of predefined spatial regions may allow to decrease the uncertainty of the propagation speed evaluation due to better statistics. In examples of the invention, a pre-defined spatial region may correspond to a pixel and/or a plurality of pixels in beamformed image data corresponding to the medium 10. Accordingly, operation (e) and optional operations (c) and (d) may be repeated in a first loop L1. As such, another spatial region in the medium 10 may be chosen in each iteration or in some iterations.

It may also be desirable to evaluate a plurality of assumed wave propagation speeds, for example to estimate an actual wave propagation speed in the medium. In an optional operation (f), the assumed wave propagation speed may be adjusted. The assumed wave propagation speed may be adjusted as a function of the figure of merit.

Operations (e) and optional operations (c), (d) and (f) may be repeated in a second loop L2. A different wave propagation speed may be assumed in each iteration and/or in some iterations.

It should be noted that various combinations of loops L1 and L2 may be possible. For example, loops L1 and L2 may performed in arbitrary order. For example, it is possible to first assume a wave propagation speed and to then perform loop L1, for example iterating operation (e) and optionally optional operations (c) and (d) for a plurality of (optionally predefined) spatial regions. Loop L2 may be performed subsequently, for example operation (e) and optional operations (c) and (d) may be iterated for a plurality of (optionally predefined) spatial regions in loop L1, then a different wave propagation speed may be assumed, for example in operation (f), for example in loop L2, and operation (e) and optional operations (c) and (d) may then be iterated again for a plurality of (optionally predefined) spatial regions in L1. Loop L2 may then be iterated for a plurality of assumed wave propagation speeds.

It is also possible to first perform operation (e) and optionally optional operations (c), (d), and (f) for a predetermined spatial region for a plurality of assumed wave propagation speeds in loop L2. Loop L1 may be performed afterwards, for example operations (e) and optional operations (c), (d), and (f) may be repeated for a different spatial region in loop L2 and for a plurality of assumed wave propagation speeds in loop L1. Loop L1 may be iterated for a plurality of spatial regions in the medium.

For example, it is possible to vary assumed wave propagation speeds in iterations while remaining at one predetermined spatial region, to then select another predetermined spatial region and to vary assumed wave propagation speeds again in iterations. It is also possible to remain at one assumed wave propagation speed, to select different spatial regions in iterations, to then assume a different wave propagation speed and to select different spatial regions again in iterations.

Various more combinations of loops L1 and L2 of the respective optional operations (c), (d), and (f) and the operation (e) are readily apparent for the skilled person. The skilled person may combine above operations as appropriate, optionally for example as a function of figures of merit, without deviating from the present disclosure.

The terms "repeat" and "iterate" may be used synonymously throughout this disclosure unless denoted differently.

In optional operation (g), an actual wave propagation speed in the medium may be estimated, optionally based on the assumed wave propagation speed having the best figure of merit and/or the best evaluation of the assumed wave propagation speeds. For example, the assumed wave propagation speed having the best figure of merit and/or the best evaluation of the assumed wave propagation speeds may be the estimated actual wave propagation speed.

An optional beamforming process may be performed after operation (g) and may be based on the propagation speed estimated in operation (g).

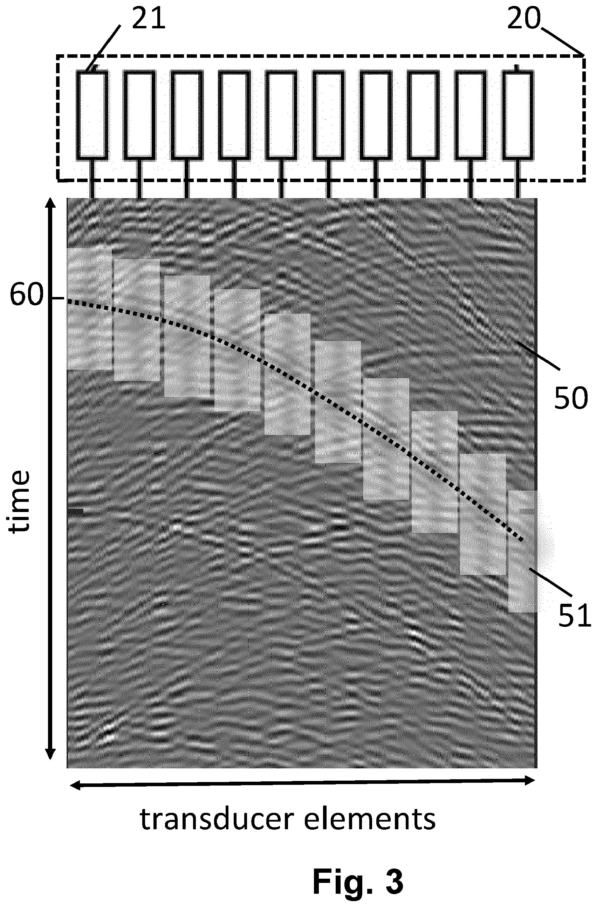

Fig. 3 shows a first example of evaluating (e) an assumed wave propagation speed based on phase properties, which may be implemented in the system of Fig. 1 and/or according to the method of Fig. 2. A plurality 20 of transducer elements 21 are displayed on the top. Spatio-temporal signal data 50 are indicated below. The spatio-temporal signal data comprise a plurality of signals respectively received by the plurality of transducer element elements 21 during a period of time. Specific subsets 51 of each signal are schematically illustrated. The subsets 51 are located along a dotted line 60. The dotted line 60 may be theoretical line and/or an imaginary line and/or may be understood as an illustration, for example as an illustration of operation (c) of determining a subset of spatio-temporal signal data as a function of a predefined spatial region 40 in the medium 10. The shape of the dotted line is related to an actual wave propagation speed in the medium. The shape of the dotted line 60 may be related to the location of a spatial region in the medium reflecting a transmitted ultrasonic pulse. The dotted line 60 may be precomputed. In other words, the sections of the signals covered by the dotted line may be associated with the spatial region. Based on said sections, a pixel of beamformed image data may be calculated in an optional beamforming operation.

Operation (c) of determining a subset of the spatio-temporal signal data as a function of a predefined spatial region in the medium and the assumed wave propagation speed may be understood as corresponding to drawing/determining/building the dotted line 60 in Fig. 3. Accordingly, by changing the assumed wave propagation speed, also the shape of the dotted line (and hence the covered signal sections) is changed. Phase properties of the respective individual signals along the dotted line may be compared to each other in the optional operation (d) of determining a figure of merit of the assumed wave propagation speed and/or the operation (e) of evaluating the assumed wave propagation speed based on phase properties of the spatio-temporal signal data.

For example, spatial temporal signal data, which may be RF data or pre-beamformed data, may comprise n signals from n transducer elements 21. Spatial temporal signal data 50 lying below (i.e. covered by) the dotted line 60 may correspond to sections of a plurality of oscillating signals, for example of the spatio-temporal signal data. Spatial temporal signal data 50 lying below the dotted line 60 may correspond to a subset of the spatio-temporal signal data 50 that may be associated with a predefined spatial region 40 in the medium 10. The subset may comprise one instance in time of a corresponding signal, for example of a signal corresponding to a respective transducer, and/or of a plurality of instances in time of a signal corresponding to a respective transducer. A figure of merit may be determined in operation (d) based on phase properties of the subset. For example, a sign proportion may be used as a figure of merit. For example, out of 100 signals corresponding to 100 respective transducers, 70 signals may exhibit a positive sign and 30 may exhibit a negative sign. A corresponding sign proportion of for example 70% may then be determined. An assumed wave propagation speed may then be evaluated based on the determined figure of merit, for example based on the sign proportion of 70%.

In other words, Fig. 3 may be interpreted as showing a dotted line 60 corresponding to a delay of signals from a predefined spatial region 40 in the medium 10, assuming an assumed wave propagation speed in the medium. An assumed wave propagation speed that is close or identical to an actual wave propagation speed in the medium 10 may result in similar and/or identical phase properties of individual signals of respective transducers 21 that correspond to areas indicated by the dotted line 60. A figure of merit may be determined based on comparing phase properties of respective individual signals corresponding to respective transducers 21.

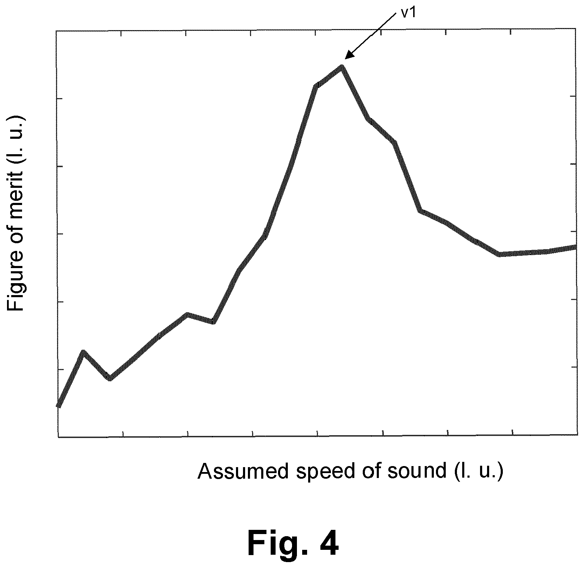

Fig. 4 shows figures of merit determined as a function of a plurality of assumed wave propagation speeds according to an example of the present disclosure. For example, a sign proportion may be used as a figure merit. The best and/or highest figure of merit may then be determined for the assumed wave propagation speed at which most signals have the same sign, i. e. the highest number of signals being either positive or negative.

For determining the figures of merit in Fig. 4, the operations described with respect to Fig. 3, i. e. for example operation (e) and optionally optional operations (c) and (d), may be repeated for a plurality of assumed wave propagation speeds. Accordingly, the imaginary/hypothetical dotted line 60 may be determined based on the plurality of assumed wave propagation speeds.

The figures of merit may be maximized for an assumed wave propagation speed that may be close and/or closest to an actual wave propagation speed, as in such case phase properties of spatio-temporal signal data may for example exhibit a highest similarity.

In the optional operation (g), an actual wave propagation speed in the medium may be estimated, optionally based on the assumed wave propagation speed having the best figure of merit, for example the best figure of merit out of a plurality of figures of merit determined during iterations of operation (d). For example, an actual wave propagation speed in the medium may be estimated by the assumed wave propagation speed v1 having the best figure of merit in Fig. 4 and/or based on the assumed wave propagation speed v1 having the best figure of merit in Fig. 4. The best figure of merit may for example be determined by interpolating between respective figures of merits as a function of assumed wave propagation speeds and selecting the maximum interpolation value.

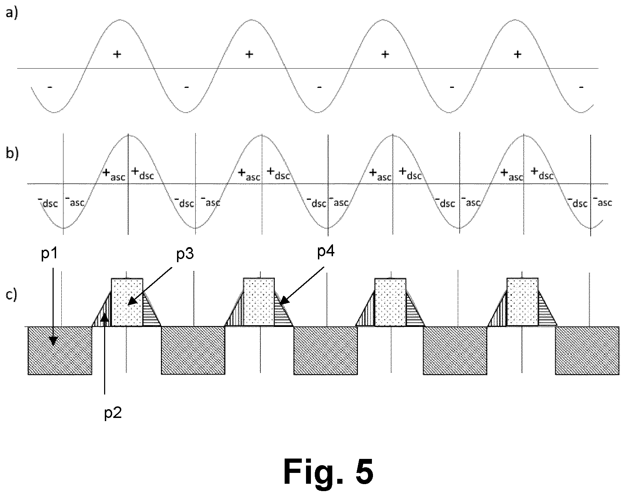

Fig. 5 shows a plurality of different phase properties of periodic signals that may be used for evaluating an assumed wave propagation speed according to examples of the present disclosure.

For example, the first diagram a) shows that signs of signal data may be used for evaluating an assumed wave propagation speed. Transducers 21 may comprise piezo-crystals and/or other objects that record and/or receive signals. Determining phase properties may require knowledge of only a single instance in time of respective signals. Thus, a fast, robust and cheap method of evaluating an assumed wave propagation speed in a medium may advantageously be provided according to examples.

The second diagram b) shows that finer categories than signs may be used. For example, positive and negative signs may be grouped according to whether the signal is ascending or descending. Corresponding categories are denoted by subscripts "asc" and "dsc" as appropriate. This may allow for a more precise evaluation of an assumed wave propagation speed and/or a more precise estimation of an actual wave propagation speed in a medium. Determining whether a signal is ascending or descending may require a knowledge of a plurality of instances in time of respective signals.

The third diagram c) shows that chosen intervals p1, p2, p3, p4 do not necessarily have to have the same size. Furthermore, they also do not need to be symmetric with respect to certain points of a signal, for example maximum/minimum points, inflection points and/or the rest position. For instance, any appropriate phase interval may be chosen. Phases of signals may be determined for example also by interpolation of recorded and/or received signals for example with periodic functions, such as sums of complex exponential functions and/or Fourier series. This may allow for an even more precise evaluation of an assumed wave propagation speed and/or a more precise estimation of an actual wave propagation speed in a medium.

According to the method of the present disclosure, a fast and automatic estimator of an actual wave propagation speed in a medium is provided. The estimation may be based on evaluating figures of merit of assumed wave propagation speeds, for examples entropies of sign proportions. Figures of merit may be determined based on spatio-temporal signal data associated with the medium. The spatio-temporal signal data may comprise per-channel samples of one or more or all pixels and/or spatial regions in beamformed image data corresponding to the medium. The figures of merit may be evaluated across the spatio-temporal signal data, for example across the per-channel samples. The figure of merit, for example an entropy of sign proprtions, may provide a quality measure of the alignment of delay wavefront which is optionally insensitive to wave shape or apodization. By minimizing entropy and/or maximizing the figure of merit, better alignment of delay wavefront may be achieved, from which an actual wave propagation speed may be estimated.

Examples of the present disclosure do not require specific types of emitted ultrasonic pulses. For example, a single planewave emission may be sufficient for carrying out optional operation (a).

Examples of the present disclosure may bear a light computational burden and may thus be used during real-time operations.

Examples of the present disclosure may advantageously be insensitive to wave shape or apodization. Example methods of the present disclosure may be performed in an automatic way.

Examples of the present disclosure may not rely on cross-correlation operators but may be based on phase properties of spatio-temporal signal data associated with the medium, for example sign proportions of spatio-temporal signal data, for example delay wave front. The computation burden may be much reduced accordingly.

Examples of the present disclosure may be insensitive to apodization, since the evaluation is based on phase properties, such as for example signs of signals, and may for example not be based on exact wave shapes.

Examples of the present disclosure may be extensible to depth-layer dependent speed estimation. Examples of the present disclosure may for example be able to work with any type of pulse transmitted into the medium during operation (a), for example focused beams.

More precise estimations of an actual wave propagation speed allow for a higher quality of beamformed image data which may be obtained by beamforming using the estimated actual wave propagation speed. A more precise estimation of an actual wave propagation speed may thus provide a higher signal-to-noise ratio (SNR) and/or a higher contrast and/or a higher spatial resolution.

Various other phase properties are known to the skilled person from the general knowledge of the skilled person. The present disclosure shall be construed as not being limited to the phase properties discussed above.

Furthermore, also various combinations of phase properties may be used. Methods described above may also be iterated, wherein different phase properties may for example be used in subsequent iterations. For example, one might start with broad categories, such as signs (cf. first diagram a), and iteratively apply finer categories, such as signs in combination with descending signals/ascending signals (cf. second diagram b) and/or appropriately chosen phase intervals (cf. third diagram c).

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

In summary the method according the present disclosure as described above provides a method and system of evaluating an assumed wave propagation speed in a medium, which advantageously is faster, more reliable, more robust, computationally less expensive and thus requires less processing power. This implies less computational costs what in particular improves a real time computation mode. Further, due to the increased preciseness a decreased variance and thus an increased reproducibility can advantageously be achieved.

## Claims

1. A method of evaluating an assumed wave propagation speed in a medium, the medium being associated with spatio-temporal signal data,
wherein the method comprises:
evaluating (e) the assumed wave propagation speed based on phase properties of the spatio-temporal signal data.

2. The method according to claim 1, wherein
the spatio-temporal signal data is or comprises pre-beamformed spatio-temporal signal data and/or radiofrequency data, and/or wherein
the spatio-temporal signal data comprises a plurality of oscillating and/or periodic signals received from the medium and/or sections of the plurality of oscillating signals.

3. The method according to claim 1 or 2, wherein
the phase properties comprise at least one:
• signs,
• phases,
• a sign proportion,
• phase intervals,
• phase evolution states, and
• proportions of phase properties.

4. The method according to any one of the preceding claims, further comprising:
determining (d) a figure of merit of the assumed wave propagation speed based on the phase properties, wherein
evaluating (e) the assumed wave propagation speed is based on the figure of merit.

5. The method according to the preceding claim, wherein the figure of merit is determined (d) by using a predefined function G,
the function G being at least one of:
a function G(x) that is symmetric about the axis x = 50%, configured to maximize at x=50% and minimize at x=0% and x=100% or to minimize at x=50% and maximize at x=0% and x=100%.
a bell-shaped function,
a normal distribution function,
a negative Shannon entropy function.

6. The method according to any one of the preceding claims, wherein
the spatio-temporal signal data are associated with a predefined spatial region in the medium.

7. The method according to any one of the preceding claims 1 to 4, further comprising:
determining (c) a subset of the spatio-temporal signal data as a function of a predefined spatial region in the medium and the assumed wave propagation speed, wherein
the assumed wave propagation speed is evaluated based on the subset.

8. The method according to the preceding claim, wherein
the subset comprises sections of the oscillating signals, wherein the sections are selected with respect to each other as a function of the spatial region and the assumed wave propagation speed.

9. The method according to any of one of the preceding claims 6 and 7, wherein the method is iterated, in parallel or in series, for a plurality of subsets, each one being associated with a different spatial region in the medium,
wherein the assumed wave propagation speed is evaluated based on figures of merit of the plurality of subsets.

10. The method according to the preceding claim, wherein
the assumed wave propagation speed is evaluated based on figures of merit of selected subsets which are above a predefined threshold, and/or
the assumed wave propagation speed is evaluated based on weighted figures of merit according to a predefined weighting function.

11. The method according to any one of the preceding claims, wherein
the method is carried out for a plurality of different assumed wave propagation speeds, and
wherein the method further comprises:
estimating (g) an actual wave propagation speed in the medium based on the assumed wave propagation speed having the best figure of merit.

12. The method according to any one of the preceding claims 1 to 10, further comprising:
adjusting (f) the assumed wave propagation speed, as a function of the figure of merit, and/or
wherein the method is iterated, such that the figure of merit is increased, wherein the method comprises in each iteration an adjusted wave propagation speed.

13. The method according to the preceding claim, wherein
evaluating (e) the assumed wave propagation speed comprises comparing the figure of merit with a predefined threshold, and/or
the method is iterated until the figure of merit is above a predefined threshold, and/or
the method further comprises: estimating (g) an actual wave propagation speed in the medium, based on the assumed wave propagation speed having the best figure of merit.

14. The method according to any of one of the preceding claims, wherein the plurality of signals is received by a respective plurality of transducer elements.

15. The method according to any of one of the preceding claims, further comprising before determining (c) the subset and/or determining (d) the figure of merit:
transmitting (a) a pulse into the medium, and
receiving (b) in response the plurality of signals from the medium.

16. A method of estimating a wave propagation speed in a medium, comprising a method according to any of one of the preceding claims 11 to 13.

17. A computer program comprising computer-readable instructions which when executed by a data processing system cause the data processing system to carry out the method according to any one of preceding method claims.

18. A system for evaluating an assumed wave propagation speed in a medium, the medium being associated with spatio-temporal signal data, wherein
the system comprises a processing unit configured to:
evaluate the assumed wave propagation speed based on the spatio-temporal signal data.
